# EUROPÄISCHE PATENTSCHRIFT

(11) **EP 3 180 013 B1**
(45) Veröffentlichungstag und Bekanntmachung des Hinweises auf die Patenterteilung: **02.04.2025**
(21) Anmeldenummer: 15744151.0
(22) Anmeldetag: 21.07.2015
(51) Int. Cl.: A61K 36/87, A61K 9/00, A61K 47/00, A61K 9/16, A61P 9/14, A61P 39/06

(54) **WASSERLÖSLICHES GRANULAT ZUR HERSTELLUNG EINER TRINKLÖSUNG**
WATER-SOLUBLE GRANULAR MATERIAL FOR PRODUCING A DRINKING SOLUTION
GRANULÉ HYDROSOLUBLE POUR LA PRÉPARATION D'UNE SOLUTION BUVABLE

(30) Priorität: 12.08.2014 DE 202014006415 U
(43) Veröffentlichungstag der Anmeldung: 21.06.2017
(73) Patentinhaber: STADA Arzneimittel AG, 61118 Bad Vilbel (DE)
(72) Erfinder: SAUERLAND, Sandra, 55216 Ingelheim am Rhein (DE); HEIGENHAUSER, Sonja, 55216 Ingelheim am Rhein (DE); SCHEURING, Uwe, 55216 Ingelheim am Rhein (DE); TIMM, Oliver, 55216 Ingelheim am Rhein (DE)
(74) Vertreter: Kernebeck, Thomas
(86) Internationale Anmeldenummer: PCT/EP2015/066659
(87) Internationale Veröffentlichungsnummer: WO 2016/023714

(56) Entgegenhaltungen:
- EP-A1- 1 550 450
- EP-A2- 2 322 158
- DATABASE WPI Week 200520, Derwent World Patents Index; AN 2005-187398, XP002744600

## Beschreibung

### HINTERGRUND DER ERFINDUNG

### 1. TECHNISCHES GEBIET

Die Erfindung betrifft ein wasserlösliches Granulat zur Herstellung einer Trinklösung gemäß Anspruch 1, insbesondere zur Vorbeugung oder Behandlung von mit leichter bis mäßiger chronischer venöser Insuffizienz der unteren Extremitäten verbundenen Beschwerden.

### 2. STAND DER TECHNIK

In dem deutschen Patent DE 600 27 481 wird eine Zusammensetzung, die in einer für die orale Verabreichung geeigneten Form ist, zur Behandlung von chronischer venöser Insuffizienz mit einem Extrakt aus Blättern von roten Weinreben vorgeschlagen.

Das deutsche Patent DE 603 32 767 beschreibt eine Filmtablette mit einem Extrakt aus roten Weinblättern.

Im EMA "Assessment Report on Vitis vinifera L., folium" (EMA/HMPC/16633/2009) vom 15. Juli 2010 werden die präklinischen und klinischen Daten bezüglich des wohl etablierten und traditionellen Einsatzes von Weinlaubblättern zusammengefasst.

Das Nahrungsergänzungsmittel "Programme Jambes Legeres" der Firma Yves Rocher ist ein Trinkgranulat, welches in neben 10 Gewichtsprozenten eines Extraktes von Hibiskus-Blüten und natürlichem Blaubeerenaroma etwa 5 Gewichtsprozente eines Extraktes von roten Weinlaubblättern enthält.

Der Gehalt an Wirksubstanz in diesem Produkt ist jedoch zu gering, um einen signifikanten Effekt bei der Vorbeugung oder Behandlung von mit leichter bis mäßiger chronischer venöser Insuffizienz der unteren Extremitäten verbundenen Beschwerden zu erzielen.

In der JP 2005/052085 A wird eine Nahrungsmittelzusammensetzung beschrieben, die einen Extrakt einer zur Gattung der Traubengewächse (Grapeaceae) gehörenden Pflanze, insbesondere *Vitis vinifera* oder *Vitis labraska,* als Wirkstoff zur Verbesserung von geschwollenen Füßen, Kälte, mit Kälte verbundenen Schmerzen oder Müdigkeit enthält.

In der EP 2 322 158 A2 wird die Verwendung von Resveratrol und/oder Traubenblattextrakt zur Aktivierung des Energiestoffwechsels beschrieben.

In der EP 1 550 450 A1 wird eine Zusammensetzung zur Vorbeugung und/oder Linderung von leichter bis mäßiger chronischer venöser Insuffizienz (CVI) der Beine beschrieben, welches als pharmakologisch aktive Substanzen einen wässrigen Extrakt aus roten Weinblättern (1) und ein die Blutzirkulation verbesserndes Mittel (2) umfasst.

Der vorliegenden Erfindung lag die Aufgabe zu Grunde ein wohlschmeckendes, wasserlösliches Granulat enthaltend ein Extraktes von roten Weinblättern zur Herstellung einer Trinklösung, die zur Vorbeugung oder Behandlung von mit leichter bis mäßiger chronischer venöser Insuffizienz der unteren Extremitäten verbundenen Beschwerden geeignet ist, aufzuzeigen.

Eine weitere Anforderung ist eine lange Lagerstabilität des Granulats ohne wesentliche Reduktion des Gehaltes der Flavonoide des Extraktes.

Außerdem sollte es von den Verwendern hinsichtlich des Geschmackes gegebenenfalls auch ohne Zusatz eines künstlichen oder natürlichen Aromastoffes akzeptiert werden, um eine hohe Compliance zu erzielen.

Überraschenderweise wurde nun gefunden, dass ein wasserlösliches Granulat zur Herstellung einer Trinklösung bestehend aus einem konzentrierten Extrakt von roten Weinblättern, einem wasserlöslichen Trägermaterial, einem Säuerungsmittel, einem Fließregulierungsmittel, einem Süßungsmittel und gegebenenfalls einem Aromastoff, wobei das wasserlösliche Trägermaterial Maltodextrin ist und das Granulat 45 bis 60 Gewichtsprozente des Extraktes von roten Weinblättern enthält, wobei die Bestandteile zusammen 100 Gewichtsprozent ergeben und wobei das Gewichtsverhältnis zwischen dem getrockneten, wässrigen Extraktes von roten Weinblättern und Maltodextrin bei 0,8:1,0 bis 1,2:1,0 ist, zur Vorbeugung oder Behandlung von mit leichter bis mäßiger chronischer venöser Insuffizienz der unteren Extremitäten verbundenen Beschwerden hervorragend geeignet ist.

Das Hauptmerkmal des Granulats liegt hierbei in der schnellen und kompletten Auflösung in heißen oder kalten Flüssigkeiten wie Wasser, Tee oder Fruchtsäften. Dies wird durch ein grobes Granulat poröser Struktur erreicht.

Weiterhin wurde überraschenderweise gefunden, dass das erfindungsgemäße Granulat eine hohe Antioxidationswirkung aufweist.

### KURZE ZUSAMMENFASSUNG DER ERFINDUNG

Gegenstand der Erfindung ist somit ein wasserlösliches Granulat zur Herstellung einer Trinklösung gemäß Anspruch 1 bestehend aus einem konzentrierten Extrakt von roten Weinblättern, einem wasserlöslichen Trägermaterial, einem Säuerungsmittel, einem Fließregulierungsmittel, einem Süßungsmittel und gegebenenfalls einem Aromastoff.

Ein weiterer Aspekt der Erfindung ist, dass sich das Granulat insbesondere zur Herstellung einer Trinklösung zur Verwendung zur Vorbeugung oder Behandlung von mit leichter bis mäßiger chronischer venöser Insuffizienz der unteren Extremitäten verbundenen Beschwerden oder zur Verbesserung des mikrovaskulären Blutflusses eignet.

Die wässrige Trinklösung ist erhältlich durch
- Mischen eines Granulats bestehend
   o aus einem konzentrierten Extrakt von roten Weinblättern, einem wasserlöslichen Trägermaterial, einem Säuerungsmittel, einem Fließregulierungsmittel, einem Süßungsmittel und gegebenenfalls einem Aromastoff,
   o wobei das wasserlösliche Trägermaterial Maltodextrin ist und das Granulat 45 bis 60 Gewichtsprozente des Extraktes von roten Weinblättern enthält, wobei die Bestandteile zusammen 100 Gewichtsprozent ergeben und das Gewichtsverhältnis zwischen dem getrockneten, wässrigen Extraktes von roten Weinblättern und Maltodextrin bei 0,8:1,0 bis 1,2:1,0 ist,
- mit Wasser oder einem wässrigen Getränk.

Ein weiterer Gegenstand der Erfindung ist eine gebrauchsfertige Verpackungseinheit bestehend im Wesentlichen aus
(A) einem oder mehreren versiegelten Portionstütchen enthaltend 500 bis 1000, vorzugsweise 700 bis 900, insbesondere etwa 800 mg eines erfindungsgemäßen Granulates; und
(B) einer Packungsbeilage, in der das Öffnen der Portionstütchen (A) und die Menge an Wasser oder eines Getränkes, in dem das Granulat aufzulösen ist, sowie gegebenenfalls die Anwendung zur Vorbeugung oder Behandlung von mit leichter bis mäßiger chronischer venöser Insuffizienz der unteren Extremitäten verbundenen Beschwerden oder zur Verbesserung des mikrovaskulären Blutflusses erläutert wird.

### DETAILLIERTE BESCHREIBUNG DER ERFINDUNG

Der Begriff "konzentrierter Extrakt von roten Weinblättern", wie er vor- und nachstehend verwendet wird, bezieht sich auf einen wässrigen Extrakt von roten Blättern der Weinrebe wie er zum Beispiel in dem vorstehend erwähnten EMA Assessment Report EMA/HMPC/16633/2009 beschrieben ist.

Dieser Extrakt ist zum Beispiel erhältlich durch
(a) Sammeln von roten Weinblättern zu einem Zeitpunkt, an dem der Flavonoidgehalt ein Optimum erreicht;
(b) Trocknen und Zerkleinern der Blätter;
(c) Schneiden der Blätter in Stücke;
(d) Extrahieren der Blätter mit Wasser bei Temperaturen von 60 bis 80°C für 6 bis 10 Stunden durch vollständige Perkolation;
(e) Konzentrieren des erhaltenen Extrakts.

Insbesondere handelt es sich dabei um den dort beschriebenen Trockenextrakt von *Vitis vinifera L.,* folium (4-6:1; Wasser).

Der Begriff "Granulat", wie er vor- und nachstehend verwendet wird, bezieht sich auf eine körnige bis pulverförmige, leicht schüttbare, feste Zusammensetzung, vorzugsweise eine grobe Zusammensetzung mit poröser Struktur. In der Regel wird es durch Granulieren erhalten, insbesondere durch Feuchtgranulierung, wobei die einzelnen Bestandteile aufgeschlämmt werden und dieses feuchte Material dann zerkleinert und getrocknet wird, oder durch Trockengranulierung bzw. Walzenkompaktierung, bei der ein Pulver der vorgemischten Bestandteile durch einen Spalt zwischen zwei Walzen getrieben wird. Die entstandenen Schülpen werden dann an einem Sieb wieder zerkleinert. Des Weiteren findet die Wirbelschichtgranulation, bei der einem Pulver der vorgemischten Bestandteile, welches sich auf einem Luftbett befindet, Wasser zugesetzt wird, Anwendung.

Schließlich kann man auch das erfindungsgemäße Granulat auch herstellen, indem man ein feuchtes Gemisch aller oder einen Teil der Bestandteile, insbesondere einem Gemisch des Extraktes und des wasserlöslichen Trägermaterials einem Sprühtrocknungsverfahren unterzieht.

Wird nur ein Teil der Bestandteile granuliert, können die anderen Komponenten später trocken dem ursprünglichen Granulat zugesetzt werden.

Bevorzugte Ausführungsformen des erfindungsgemäßen Granulats sind solche, wobei:
(i) es 45 bis 60 Gewichtsprozente eines getrockneten, wässrigen Extraktes von roten Weinblättern, insbesondere den Trockenextrakt von Vitis vinifera L., folium (4-6: 1; Wasser) gemäß der EMA/HMPC/ 16633/2009 enthält; besonders bevorzugt enthält das Granulat bezogen auf eine Einzelportion, zum Beispiel eines Pulvertütchen (Sachet), 300 bis 500 mg, vorzugsweise 320 bis 400 mg, insbesondere 355 bis 380 mg, meist bevorzugt etwa 360 mg des Trockenextrakts von Vitis vinifera L., folium (4-6: 1; Wasser) gemäß der EMA/HMPC/16633/2009;
(ii) das wasserlösliche Trägermaterial Maltodextrin ist, wobei das Gewichtsverhältnis zwischen dem getrockneten, wässrigen Extraktes von roten Weinblättern und dem wasserlöslichen Trägermaterial bei 0,8 zu 1,0 bis 1,2 zu 1,0 liegt;
(iii) das Säuerungsmittel eine organische Säure ausgewählt aus der Gruppe bestehend aus Äpfelsäure, Zitronensäure, Ascorbinsäure, Milchsäure und Weinsäure, insbesondere Weinsäure ist. Vorzugsweise liegt der pH- Wert der resultierenden Trinklösung bei Auflösung in neutralem Wasser unterhalb von pH 6, insbesondere bei pH 3,0 bis pH 5,5;
(iv) das Fließregulierungsmittel kolloides Siliziumdioxid ist;
(v) das Süßungsmittel ein Süßstoff ausgewählt aus der Gruppe bestehend aus Acesulfam, Arpartam, Cyclamat, Neohesperidin, Saccharin, Sucralose, Steviosid und Thaumatin, insbesondere Sucralose ist;
Neben den genannten Bestandteilen kann das Granulat auch einen oder mehrere künstliche oder natürliche Aromastoffe enthalten. Bevorzugt sind Kräuter-, Tee- und Fruchtaromen, insbesondere in fester Form. Bevorzugt sind folgende Aromen: Pfefferminze, Tee, Weintraube, Brombeere, Himbeere, Heidelbeere, Walderdbeere, Kirsche, Schwarze Johannisbeere, Pflaume, Preiselbeere Grenadine und Limette, insbesondere Limette. Ganz besonders bevorzugt ist das erfindungsgemäße Granulat frei von zusätzlichen Aromastoffen.
Offenbart wird auch ein Granulat im Wesentlichen bestehend aus 20 bis 80 Gew.-% des getrockneten, wässrigen Extraktes von roten Weinblättern, 20 bis 80 Gew.-% eines wasserlöslichen Trägermaterials, 8 bis 20 Gew.-% eines Säuerungsmittels, 0,5 bis 1,5 Gew.-% eines Fließregulierungsmittels, 0,2 bis 0,8 Gew.-% eines Süßungsmittels und gegebenenfalls bis zu 2,0 Gew.-% eines Aromastoffes, wobei diese Bestandteile zusammen 100 Gewichtsprozente ergeben.

(vi) Offenbart wird ferner ein Granulat im Wesentlichen besteht aus folgenden Bestandteilen:

| Gehalt in Gew.-% | Bestandteil |
|---|---|
| 20 - 80 | konzentrierten Extrakt von roten Weinblättern |
| 20 - 80 | Maltodextrin |
| 8 - 20 | Weinsäure |
| 0,5 - 1,5 | Wasserfreies kolloidales Siliziumdioxid |
| 0,2 - 0,8 | Sucralose |
| 0 - 2,0 | Limettenaroma |

wobei diese Bestandteile zusammen 100 Gewichtsprozente ergeben;
(vii) es zur Herstellung einer Trinklösung zur Vorbeugung oder Behandlung von mit leichter bis mäßiger chronischer venöser Insuffizienz der unteren Extremitäten verbundenen Beschwerden oder zur Verbesserung des mikrovaskulären Blutflusses verwendet wird;
(viii) es zur Herstellung eines Nahrungsergänzungsmittels in Form einer Trinklösung zur Verbesserung des Stoffwechsels durch Verringerung von freien Radikalen.

Vorzugsweise wird das erfindungsgemäße Trinkgranulat in portionsgerechten Pulvertütchen (Sachets) abgefüllt. In der Regel enthalten diese Sachets 500 bis 1000, vorzugsweise 700 bis 900, insbesondere etwa 800 mg des erfindungsgemäßen Granulates. Sie werden aus unbeschichtetem oder beschichtetem Aluminiumlaminat, insbesondere mit Surlyn^{®} der Fa. DuPont beschichtetem Aluminiumlaminat gefertigt und weisen eine Länge von 5,0 bis 10,0 cm, vorzugsweise 6,0 bis 8,0, insbesondere etwa 7,5 cm sowie eine Breite von 1,0 bis 3,0 cm, vorzugsweise 1,5 bis 2,5, insbesondere etwa 2,0 cm auf.

Weiterhin verfügen sie an einem Ende über eine Einkerbung, die das Öffnen der Sachets erleichtert.

### Beispiel 1

Es wird ein Granulat hergestellt und in Pulvertütchen abgefüllt, wobei jedes Pulvertütchen ein Granulat bestehend aus folgenden Bestandteilen enthält:

| Bestandteil | Gehalt in mg |
|---|---|
| konzentrierten Extrakt von roten Weinblättern (4-6:1; Wasser) | 360 |
| Maltodextrin | 360 |
| Weinsäure | 75 |
| Wasserfreies kolloidales Siliziumdioxid | 8 |
| Sucralose | 5 |

Dazu wird die entsprechende Menge des Maltodextrins in kleinen Portionen zu dem Dick- Extrakt von roten Weinlaubblättern gegeben und miteinander gemischt. Die feuchte Mischung wird in einem Sprühtrockner zu einem trockenen Granulat versprüht. Anschließend wird das Granulat mit den übrigen Bestandteilen gemischt und abgefüllt.

Das in einem Pulvertütchen enthaltene Trinkgranulat löst sich innerhalb von 30 bis 40 sec. in 100-200 ml heisser oder kalter Flüssigkeit wie Wasser, Tee oder Fruchtsaft auf. Die erhaltene Trinklösung wurde in einem in Italien durchgeführten Konsumententest von allen Probanden als geschmacklich angenehm und wohlschmeckend empfunden und eignet sich hervorragend die mit leichter bis mäßiger chronischer venöser Insuffizienz der unteren Extremitäten verbundenen Beschwerden zu lindern.

### Beispiel 2

Es wird ein Granulat hergestellt und in Pulvertütchen abgefüllt, wobei jedes Pulvertütchen ein Granulat bestehend aus folgenden Bestandteilen enthält:

| Bestandteil | Gehalt in mg |
|---|---|
| konzentrierten Extrakt von roten Weinblättern (4-6:1; Wasser) | 360 |
| Maltodextrin | 360 |
| Weinsäure | 75 |
| Wasserfreies kolloidales Siliziumdioxid | 8 |
| Sucralose | 5 |
| Limettenaroma | 8 |

Dazu wird die entsprechende Menge des Maltodextrins in kleinen Portionen zu dem Dick- Extrakt von roten Weinlaubblättern gegeben und miteinander gemischt. Die feuchte Mischung wird in einem Sprühtrockner zu einem trockenen Granulat versprüht. Anschließend wird das Granulat mit den übrigen Bestandteilen gemischt und abgefüllt.

Das in einem Pulvertütchen enthaltene Trinkgranulat löst sich schnell in 100-200 ml heißer oder kalter Flüssigkeit wie Wasser, Tee oder Fruchtsaft auf. Die erhaltene Trinklösung wurde in einem in Italien durchgeführten Konsumententest von allen Probanden als geschmacklich sehr angenehm und wohlschmeckend empfunden und eignet sich hervorragend die mit leichter bis mäßiger chronischer venöser Insuffizienz der unteren Extremitäten verbundenen Beschwerden zu lindern.

### Beispiel 3

### Konsumententest

202 Probanden erhielten das erfindungsgemäße Trinkgranulat (102 erhielten Granulat gemäß Beispiel 1, 100 erhielten Granulat gemäß Beispiel 2) und wurden nach der Verkostung befragt, inwieweit Ihnen der Geschmack des resultierenden Getränkes gefalle. Dabei wurden die folgenden Ergebnisse erzielt:

| Geschmack | Beispiel 1 | Beispiel 2 |
|---|---|---|
| Sehr gut | 13 | 14 |
| Gut | 70 | 71 |
| Weder gut noch schlecht | 14 | 12 |
| Schlecht | 4 | 2 |
| Sehr schlecht | 1 | 1 |

### Beispiel 4

### Untersuchung zur antioxidativen Wirkung

Der ORAC(=Oxygen Radical Absorbance Capacity) -Test ist ein standardisiertes Verfahren zur Bestimmung der Fähigkeit zum Abfangen von Sauerstoffradikalen von Naturstoffen und wird in Trolox-Äquivalenten (µmol TE/100 g) angegeben. Das Messprinzip ist eine Messung der Fluoreszenz des Farbstoffs Fluorescein bis zu dessen Erschöpfung durch einen künstlichen Radikalenerzeuger. Ein zugegebenes Antioxidans verzögert die Oxidation des Fluoresceins und je höher die Kapazität ist, desto grösser ist die gemessene Fläche unter der Zeitkurve (AUC). Eine Beschreibung findet sich in zum Beispiel in Wikipedia unter http
://www.en.wikipedia.org/wiki/Oxygen_radical_absorbance_capacit y.

Für die Durchführung der ORAC-Tests wurde das hierfür akkreditierte Institut Prof. Kurz GmbH in 50933 Köln beauftragt (www.institut-kurz.de) und führte die Messungen mittels fluorimetrischen Mikrotiterplattentests IK2002-hydrophil durch.

In einer Testreihe im März 2015 wurde das erfindungsgemäße Granulat mit 360 mg Wirkprinzip gegenüber anderen handelsüblichen Fertigprodukten getestet. Die Ergebnisse sind in der folgenden Tabelle wiedergeben:

| Produkt | ORAC-Wert [µmol TE/100 g] |
|---|---|
| Erfindungsgemäßes Granulat (Charge 1) | 170500 |
| Erfindungsgemäßes Granulat (Charge 2) | 177900 |
| Pycnogenol^{®} (Pinienrindenextrakt) 50 mg GPH Kapseln, Hecht-Pharma GmbH, PZN 09188092 | 161700 |
| Vitamin C 200 mg Tabletten, medphano Arzneimittel GmbH, PZN 04588935 | 108000 |

Der ORAC-Wert der beiden erfindungsgemäßen Proben liegt in derselben Größenordnung und ist sogar noch ein wenig höher als der von Pycnogenol^{®}, das als eines der am besten untersuchten Antioxidantien gilt. Vitamin C, das ebenfalls als Antioxidans verwendet wird, hat einen um ca. 40% niedrigeren ORAC-Wert.

## Patentansprüche

1. Ein wasserlösliches Granulat zur Herstellung einer Trinklösung bestehend aus einem konzentrierten Extrakt von roten Weinblättern, einem wasserlöslichen Trägermaterial, einem Säuerungsmittel, einem Fließregulierungsmittel, einem Süßungsmittel und gegebenenfalls einem Aromastoff, **dadurch gekennzeichnet, dass** das wasserlösliche Trägermaterial Maltodextrin ist und dass das Granulat 45 bis 60 Gewichtsprozente des Extraktes von roten Weinblättern enthält, wobei die Bestandteile zusammen 100 Gewichtsprozent ergeben und dass das Gewichtsverhältnis zwischen dem getrockneten, wässrigen Extraktes von roten Weinblättern und Maltodextrin bei 0,8:1,0 bis 1,2:1,0 ist.

2. Ein Granulat nach Anspruch 1, **dadurch gekennzeichnet, dass** der Extrakt von roten Weinblättern erhältlich ist durch ein Verfahren, welches die folgenden Schritte umfasst:
(a) Sammeln von roten Weinblättern zu einem Zeitpunkt, an dem der Flavonoidgehalt ein Optimum erreicht;
(b) Trocknen und Zerkleinern der Blätter;
(c) Schneiden der Blätter in Stücke;
(d) Extrahieren der Blätter mit Wasser bei Temperaturen von 60 bis 80°C für 6 bis 10 Stunden durch vollständige Perkolation;
(e) Konzentrieren des erhaltenen Extrakts.

3. Ein Granulat nach einem der Ansprüche 1 oder 2, **dadurch gekennzeichnet, dass** das Säuerungsmittel eine organische Säure ausgewählt aus der Gruppe bestehend aus Äpfelsäure, Zitronensäure, Ascorbinsäure, Milchsäure und Weinsäure, insbesondere Weinsäure ist.

4. Ein Granulat nach einem der Ansprüche 1 bis 3, **dadurch gekennzeichnet, dass** das das Fließregulierungsmittel kolloides Siliziumdioxid ist.

5. Ein Granulat nach einem der Ansprüche 1 bis 4, **dadurch gekennzeichnet, dass** das Süßungsmittel ein Süßstoff ausgewählt aus der Gruppe bestehend aus Acesulfam, Arpartam, Cyclamat, Neohesperidin, Saccharin, Sucralose, Steviosid und Thaumatin, insbesondere Sucralose ist.

6. Granulat nach einem der Ansprüche 1 bis 5 zur Herstellung einer Trinklösung zur Verwendung zur Vorbeugung oder Behandlung von mit leichter bis mäßiger chronischer venöser Insuffizienz der unteren Extremitäten verbundenen Beschwerden oder zur Verwendung zur Verbesserung des mikrovaskulären Blutflusses.

7. Eine gebrauchsfertige Verpackungseinheit bestehend im Wesentlichen aus
(A) einem oder mehreren versiegelten Portionstütchen enthaltend 500 bis 1000, vorzugsweise 700 bis 900, insbesondere etwa 800 mg eines Granulates gemäß eines oder mehrerer der Ansprüche 1 bis 6; und
(B) einer Packungsbeilage, in der das Öffnen der Portionstütchen (A) und die Menge an Wasser oder eines Getränkes, in dem das Granulat aufzulösen ist, sowie gegebenenfalls die Anwendung zur Vorbeugung oder Behandlung von mit leichter bis mäßiger chronischer venöser Insuffizienz der unteren Extremitäten verbundenen Beschwerden oder zur Verbesserung des mikrovaskulären Blutflusses erläutert wird.

## Claims

1. Water-soluble granules for the preparation of a drinking solution consisting of a concentrated extract of red vine leaves, a water-soluble carrier material, an acidifying agent, a flow regulator, a sweetening agent and optionally a flavouring, **characterized in that** the water-soluble carrier material is maltodextrin and **in that** the granules contain 45 to 60 percent by weight of the extract of red vine leaves, where the constituents together amount to 100 percent by weight, and **in that** the weight ratio between the dried, aqueous extract of red vine leaves and maltodextrin is 0.8:1.0 to 1.2:1.0.

2. Granules according to Claim 1, **characterized in that** the extract of red vine leaves is obtainable by a process comprising the following steps:
(a) collection of red vine leaves at a time when the flavonoid content reaches an optimum;
(b) drying and crushing of the leaves;
(c) cutting of the leaves into pieces;
(d) extraction of the leaves with water at temperatures of 60°C to 80°C for 6 to 10 hours by complete percolation;
(e) concentration of the extract obtained.

3. Granules according to either of Claims 1 and 2, **characterized in that** the acidifying agent is an organic acid selected from the group consisting of malic acid, citric acid, ascorbic acid, lactic acid and tartaric acid, in particular tartaric acid.

4. Granules according to any of Claims 1 to 3, **characterized in that** the flow regulator is colloidal silicon dioxide.

5. Granules according to any of Claims 1 to 4, **characterized in that** the sweetening agent is a sweetener selected from the group consisting of acesulfame, aspartame, cyclamate, neohesperidin, saccharin, sucralose, stevioside and thaumatin, in particular sucralose.

6. Granules according to any of Claims 1 to 5 for the preparation of a drinking solution for use for preventing or treating symptoms associated with mild to moderate chronic venous insufficiency of the lower extremities or for use for improving microvascular blood flow.

7. Ready-to-use packaging unit consisting essentially of
(A) one or more sealed sachets containing 500 to 1000, preferably 700 to 900, in particular about 800 mg of granules according to one or more of Claims 1 to 6; and
(B) a package leaflet explaining the opening of the sachets (A) and the amount of water or of a drink in which the granules are to be dissolved, and optionally the use for preventing or treating symptoms associated with mild to moderate chronic venous insufficiency of the lower extremities or for improving microvascular blood flow.

## Revendications

1. Granulés hydrosolubles permettant de préparer une solution buvable, composés d'un extrait concentré de feuilles de vigne rouge, d'un matériau support hydrosoluble, d'un acidifiant, d'un agent de régulation d'écoulement, d'un édulcorant et éventuellement d'une substance aromatique, **caractérisés en ce que** le matériau support hydrosoluble est de la maltodextrine et **en ce que** les granulés contiennent 45 à 60 % en poids de l'extrait de feuilles de vigne rouge, où les composants constituent ensemble 100 % en poids, et **en ce que** le rapport pondéral entre l'extrait aqueux séché de feuilles de vigne rouge et la maltodextrine est compris entre 0,8:1,0 et 1,2:1,0.

2. Granulés selon la revendication 1, **caractérisés en ce que** l'extrait de feuilles de vigne rouge est obtenu par un procédé comprenant les étapes ci-après consistant à :
(a) collecter des feuilles de vigne rouge à un moment où la teneur en flavonoïdes atteint un niveau optimal ;
(b) sécher et broyer les feuilles ;
(c) couper les feuilles en morceaux ;
(d) obtenir l'extrait à partir des feuilles, avec de l'eau, à des températures comprises entre 60 et 80 °C pendant 6 à 10 heures, par percolation complète ;
(e) concentrer l'extrait obtenu.

3. Granulés selon l'une des revendications 1 ou 2, **caractérisés en ce que** l'acidifiant est un acide organique choisi dans le groupe composé de l'acide malique, l'acide citrique, l'acide ascorbique, l'acide lactique et l'acide tartrique, en particulier l'acide tartrique.

4. Granulés selon l'une des revendications 1 à 3, **caractérisés en ce que** l'agent de régulation d'écoulement est du dioxyde de silicium colloïdal.

5. Granulés selon l'une des revendications 1 à 4, **caractérisés en ce que** l'édulcorant est un édulcorant choisi dans le groupe composé de l'acésulfame, l'aspartame, le cyclamate, la néohespéridine, la saccharine, le sucralose, le stévioside et la thaumatine, en particulier le sucralose.

6. Granulés selon l'une des revendications 1 à 5, permettant de préparer une solution buvable destinée à être utilisée pour prévenir ou traiter des troubles associés à une insuffisance veineuse chronique légère à modérée des extrémités inférieures ou pour améliorer la circulation sanguine microvasculaire.

7. Unité de conditionnement prête à l'emploi, constituée essentiellement de
(A) un ou plusieurs sachets-portions scellés contenant 500 à 1000 mg, de préférence 700 à 900 mg, en particulier environ 800 mg de granulés selon une ou plusieurs des revendications 1 à 6 ; et
(B) une notice expliquant l'ouverture des sachets-portions (A) et la quantité d'eau ou de boisson dans laquelle les granulés doivent être dissous, et éventuellement l'utilisation pour prévenir ou traiter des troubles associés à une insuffisance veineuse chronique légère à modérée des extrémités inférieures ou pour améliorer la circulation sanguine microvasculaire.
